(19) Europäisches Patentamt
European Patent Office
Office européen des brevets

(11) Publication number: **0 288 255 B1**

(12) **EUROPEAN PATENT SPECIFICATION**

(45) Date of publication of patent specification: **13.07.94**

(51) Int. Cl.5: **C12P 9/00**, C07F 9/10

(21) Application number: **88303542.0**

(22) Date of filing: **20.04.88**

# (54) Method of producing phospholipid derivatives.

(30) Priority: **21.04.87 JP 96243/87**

(43) Date of publication of application:
**26.10.88 Bulletin 88/43**

(45) Publication of the grant of the patent:
**13.07.94 Bulletin 94/28**

(84) Designated Contracting States:
**CH DE FR GB IT LI NL SE**

(56) References cited:
**DE-A- 2 717 547**
**US-A- 2 052 029**

**CHEM. PHYS. LIPIDS, 1969, North-Holland Publ. Co., Amsterdam(NL); K. INOUE et al., pp. 70-77&NUM;**

**CANADIAN JOURNAL OF BIOCHEMISTRY, vol. 51, 1973; pp. 747-753&NUM;**

**METHODS IN ENZYMOLOGY, vol. XIV, Lipids, J.M. Lowenstein (ed.), Academic Press, New York, NY (US), 1969; pp. 197-203&NUM;**

**COURS DE BIOCHIMIE, Lipides, Edgar Lederer, Edisciences, Paris (FR), 1970; pp. 98-105&NUM;**

(73) Proprietor: **KABUSHIKI KAISHA YAKULT HONSHA**
**1-19, Higashishinbashi 1-chome**
**Minato-ku Tokyo 105(JP)**

Proprietor: **KABUSHIKI KAISHA NIIGATA TEKKOSHO**
**4-1, Kasumigaseki 1-chome**
**Chiyoda-ku Tokyo 100(JP)**

(72) Inventor: **Kudo, Satoshi**
**K.K. Yakuruto Honsha,**
**1-1-19, Higashishinbashi**
**Minato-ku, Tokyo(JP)**
Inventor: **Sawada, Haruji**
**K.K. Yakuruto Honsha,**
**1-1-19, Higashishinbashi**
**Minato-ku, Tokyo(JP)**
Inventor: **Watanabe, Tsunekazu**
**K.K. Yakuruto Honsha,**
**1-1-19, Higashishinbashi**
**Minato-ku, Tokyo(JP)**
Inventor: **Kuroda, Akio**
**K.K. Yakuruto Honsha,**
**1-1-19, Higashishinbashi**
**Minato-ku, Tokyo(JP)**

**EP 0 288 255 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

(74) Representative: **Sanderson, Laurence Andrew et al**
**SANDERSON & CO.**
**European Patent Attorneys**
**34, East Stockwell Street**
**Colchester Essex CO1 1ST (GB)**

## Description

The present invention relates to a method of producing phospholipid derivatives.

Lecithins such as soybean lecithin and egg yolk lecithin are well-known, and utilizing their surface activity and physiological activity they have been widely used as raw materials in the manufacture of foods, cosmetics, paints, lubricants, magnetic materials, animal feeds and agricultural chemicals. We are here concerned with a largely new class of phospholipid derivatives, produced by causing enzymes having a transphosphatidylation action to act on existing phospholipid derivatives together with compounds containing an hydroxyl group - this largely new class of phospholipid derivatives being useful substances in many cases having advantageous properties as compared with the well-known raw material phospholipid derivatives. This invention relates more specifically to a method of producing this class of relatively new phospholipid derivatives, having two phosphatidyl groups, from known raw material phospholipid derivatives, by the transphophatidylation reaction.

Diphosphatidylglycerol (also often called cardiolipin) is a known phospholipid derivative having two phosphatidyl groups in its molecular structure. It is widely distributed in animals, plants and bacteria, and the usual practice for obtaining this phospholipid derivative has therefore been to extract it from a natural substance containing it, such as the heart muscle or cerebrum of cattle, or the cell of corynebacterium or brevibactrium. This known extractive method is not however an advantageous one, since no matter what raw material is used its content of the desired phospholipid derivative is so low that isolating the desired substance from the extract is very tedious. In any case, this kind of extractive method cannot serve as a means for mass production, because of the limitation on the amount of raw material available. There is an alternative known method in which one molecule of disphosphatidylglycerol is produced from two molecules of phosphatidylglycerol by utilizing the transphosphatidylation activity of phospholipase D (Biochim. biophys. acta, 210, 350, 1970) but the utility of this method is restricted from the standpoint of the availability of the raw material, in that there is no reliable source of phosphatidylglycerol.

In any event, there is no established method for synthesizing, as occasion demands, any phospholipid derivative having two phosphatidyl residues of this type, apart from diphosphatidylglycerol itself.

It has been drawn to our attention that a paper by N.Z.Stanacev entitled "Enzymatic Formation of Cardiolipin from Phosphatidylglycerol by the Transphosphatidylation Mechanism catalyzed by Phospholipase D" in Canadian Journal of Biochemistry, Vol.51, (1973), pages 747-753 describes a method for synthesizing cardiolipin from two molecules of phosphatidylglycerol by enzyme reaction. However this Stanacev method, even if it was otherwise practical, is effectively restricted to the production of cardiolipin itself, because the procedure could be applied to the production of other phospholipid derivatives having two phosphatidyl residues only if one were first to synthesize a suitably-substituted phosphatidylglyceroid for use as starting material - a need which of course makes the overall procedure quite impractical.

We have however now found that it is possible to provide a method of producing a phospholipid derivative having two phosphatidyl residues, using a readily available, inexpensive phospholipid derivative a starting material - and indeed thereby to produce such phospholipid derivatives having two phosphatidyl residues besides just diphosphatidylglycerol itself.

According to this invention there is provided a method of producing a phospholipid derivative having two phosphatidyl residues in its molecule, in which a phospholipid derivative of general formula:

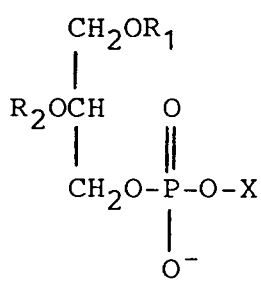

(I)

(in which $R_1$ and $R_2$, which may be the same or different, each represents an alkanoyl or an alkyl group, and X represents an organic residue derived by removal of one hydroxyl group from an hydroxyl-group-containing compound) is reacted with a polyhydric alcohol of general formula:

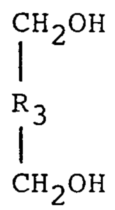

(II)

(in which $R_3$ represents an optionally-substituted alkylene group) in the presence of phospholipase D having transphosphatidylation activity (E.C. 3.1.4.4), to produce a phospholipid derivative of general formula:

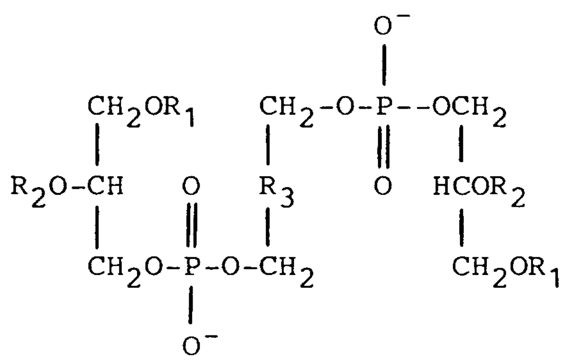

(III)

(in which $R_1$, $R_2$, $R_3$ and X all have the previously-stated meanings).

The alkanoyl and/or alkyl group(s) $R_1$ and/or $R_2$ each advantageously contains from 1 to 6 carbon atoms, and preferably from 1 to 4 carbon atoms.

The alkylene group $R_3$ advantageously contains from 1 to 6 and preferably from 1 to 4 carbon atoms, and is optionally substituted with an hydroxyl group or with an alkoxy or hydroxy-alkyl group. The alkoxy or hydroxy-alkyl substituents on the alkylene group $R_3$ advantageously contain from 1 to 6 carbon atoms, and preferably from 1 to 4 carbon atoms.

The phospholipid derivative (I) used as starting material can desirably be a natural lecithin, a phospholipid derivative fractioned from said natural lecithin, or a synthetic phospholipid derivative. The natural lecithin is conveniently soybean lecithin, egg-yolk lecithin or rape-seed lecithin.

The polyhydric alcohol (II) advantageously is glycerin, erythritol, adonitol, mannitol, sorbitol, sedoheptylol and/or 2-hydroxymethyl-1,3-propanediol.

The phospholipid derivative of general formula I is conveniently brought to reaction suspended in water, and the phospholipase D is usually introduced into a mixture of the other reactants and incubated therewith, if desired with agitation.

The transphosphatidylation activity of the phospholipase D is very desirably promoted by the presence of calcium ions, and it is recommended that the calcium ion should be present at a concentration of from 1 to 100 mM, desirably in the range of from 4 to 30 mM.

The molar amount of polyhydric alcohol (II) employed should be less than 50 times the molar amount of phospholipid derivative (I), and desirably in the range of from 2 to 40 times the molar amount of phospholipid derivative (I).

The phospholipase D employed is advantageously one derived from a streptomyces, and the reaction is desirably effected at a temperature in the range of from 20° to 60°C, and at a pH greater than 4. In most cases the reaction is best effected at a temperature of from 35° to 50°C, and at a pH of from 5 to 8.4.

It has been found advantageous to add an amount of organic solvent to the reaction system sufficient to facilitate the dispersion of the phospholipid derivative (I) and active phospholipase D and thereby increase the reaction efficiency, and in our experience the organic solvent preferably is or includes ethyl acetate added in an amount of from 1% to 6% (v/v).

It may here be noted that after the above-indicated enzyme reaction has occurred the resultant reaction mixture also includes the compound of the following general formula:

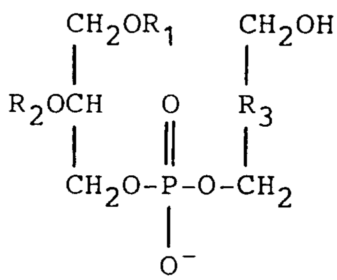 (IV)

(wherein $R_1$, $R_2$ and $R_3$ have the same meanings as these indicated elsewhere) in which one phosphatidyl group of the phospholipid derivative of the general formula (I) has been transferred to the polyhydric alcohol of the general formula (II).

We therefore consider that the overall production method of this invention for producing the desired phospholipid derivative (III) is to be regarded as a two-stage transphosphatidylation reaction, in which first the compound of the general formula (IV) is formed and then a second transphosphatidylation takes place in which the resulting compound having an alcoholic hydroxyl group at the end of its molecule serves as a phosphatidyl residue acceptor.

It may also here be noted that in the already well-known transphosphatidylation reaction which will produce a new phospholipid derivative having a single phosphatidyl group starting from a phospholipid derivative of the same type as the compounds of general formula (I) type and a hydroxyl group bearing compound, the usual practice is to effect that reaction using a greatly excessive amount of the hydroxyl group containing compound (of over 100 times the molar amount of the phospholipid derivative) in order to push the equilibrium towards the formation of a phospholipid derivative (III) and suppress the hydrolysis reaction.

On the contrary, however, in the method of this invention for producing phospholipid derivative having two phosphatidyl residues by the above-mentioned two-stage transfer reaction, the use of any excessive amount of the hydroxyl group containing compound or the polyhydric alcohol of the general formula (II) must be restricted.

We consider that the reason for this is to be found in the possibility that, besides the second transphosphatidylation, there can also take place another balanced reaction, as follows:

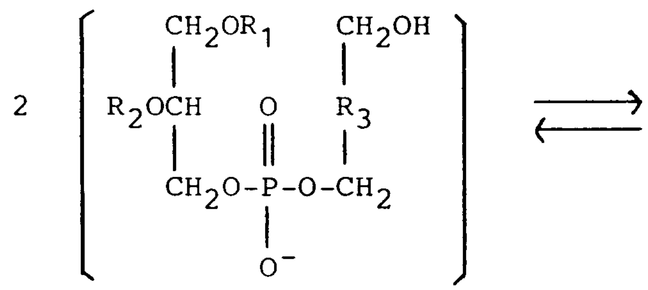

(IV)

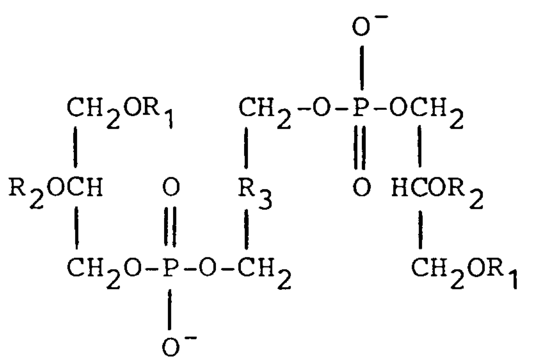

+

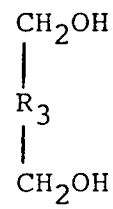

(III) (II)

(wherein $R_1$ ,$R_2$ and $R_3$ have the same meanings as those indicated elsewhere) in which the polyhydric alcohol of the general formula (II) participates, and therefore the production of the phospholipid derivative (III) is suppressed with increase in amount of the polyhydric alcohol (II).

The nature of the phospholipid derivative (I) and the polynydric alcohol (II) which are used as starting material for the production of the phospholipid derivative (III) according to the production method of this invention have to be selected having regard to the structure of the desired product, and are not otherwise restricted. However, specific examples of raw materials suitable for use as such starting materials may be cited as follows. The raw material phospholipid derivatives (I) may be soybean lecithin, egg yolk lecithin, rape-seed lecithin, fractionated lecithin derived from any of these natural lecithins, as well as synthetic phospholipid derivatives or the like; while the polyhydric alcohol (II) may be glycerin, erythritol, adonitol, mannitol, sorbitol, sedoheptytol or 2-hydroxymethyl-1,3-propanediol, etc.

While the phospholipases D used for bringing about transphosphatidylation may be any of those derived from animals, plants and microorganisms, we have found that one derived from a microoganism, and particularly one derived from an actinomycete, is generally best from the standpoint of activity and economy, and this can be recommended as a suitable enzyme for use in the production method of the invention.

A typical procedure for bringing about the two-stage transphosphatidylation from the previously mentioned raw material is as follows.

After the raw material phospholipid derivative (I) has been suspended in water, the polyhydric alcohol (II) is added followed by addition of a phospholipase D and calcium ion for activating it and thus causing the phospholipase D to exhibit transphosphatidylation activity) and thereupon performing incubation. The amount of the polyhydric alcohol (II) added should preferably be less than 50 times the molar amount of the phospholipid derivative, for the previously-mentioned reasons and a suitable amount is usually from 2 to 40 times the molar amount. The phospholipid derivative concentration can be chosen as desired, provided that the suspension can be agitated. The calcium ion concentration should be from 1 to 100 mM, and is preferably from 4 to 30 mM. The reaction temperature and pH are determined in accordance with the characteristics of the enzyme used. where the phospholipase D used is one derived from a streptomyces, the preferred temperature is between 20 and 60 °C (more preferably 35 to 50 °C), and a suitable pH is

above 4 (more preferably 5 to 8.4).

A small amount of organic solvent can be added to the reaction system to facilitate the dispersion of the phospholipid derivative and active phospholipase D, and thereby to increase the reaction efficiency. Effective organic solvents include ethyl acetate, n-propyl n-propionate, 4-heptanone, isobutyl ketone, pentane, hexane, benzene, xylene and chloroform, etc. If however the amount of such organic solvent added is increased too much, the second-stage transphosphatidylation tends to become impeded, although the first-stage transphosphatidylation is accelerated. In the case of ethyl acetate, for example, while the addition of 1 to 2 % (v/v) produces a good result, the addition of more than about 6% (v/v) results in a decreased yield of the product.

Any suitable separation and purification methods can be used to extract the desired phospholipid derivative (III) from the reaction mixture, such as solvent separation methods employing acetone, ethanol or the like, column chromatography employing silica gel, alumina gel, reverse phase carrier or the like, or thin layer chromatography.

Phospholipid derivatives having two phosphatidyl residues can be easily produced in accordance with the present invention, by one-step and simple but efficient procedures, using as raw material phospholipid derivatives which are easily and inexpensively available. Although a method of producing cardiolipin, which is one particular phospholipid derivative having two phosphatidyl residues, from two molecules of phosphatidylglycerol by the enzyme reaction has previously been available, the phosphatidylglycerol raw material for that procedure has not been easily available, so that in practice that method has had to start from the synthesis of phosphatidylglycerol. The present invention however has very great utility, in that it provides a general and simple method of production by which it is possible to synthesize not only cardiolipin but also a large number of other phospholipid derivatives having two phosphatidyl residues.

## EXAMPLES

The present invention will now be described in greater detail by means of the following examples.

### Example 1

A mixture of 1 to 20 mg of a polyhydric alcohol of the general formula (II), 20 $\mu$l 20 mM of 20 mM acetate buffer (pH 5.5), 1 $\mu$l of 500 mM $CaCl_2$ solution, 5 $\mu$l of ethyl acetate, 5 $\mu$l of an enzyme solution containing 0.24 units of actinomycete-derived phospholipase D, and 2 mg of a commercial soybean lecithin (Unimills Inc.) containing 80% (w/w) of phosphatidylcholine, was incubated at 50 °C for 60 minutes while shaking the reaction mixture. After the reaction had been completed, the desired substance was extracted with chloroform/methanol (2/1) and then the extract was developed by a silica-gel thin layer chromatography. A phosphorus detecting reagent was sprayed onto the developed extract, thereby subjecting it to quantitative analysis.

The following Table 1 shows the results (percentages of the products and unreacted phosphatidylcholine) obtained by using, as the polyhydric alcohol (II), respectively glycerin, erythritol, sorbitol and mannitol, in the same exemplary manner as in Example 1.

Table 1

| Polyhydric Alcohol addition | Glycerin | | | | * | Sorbitol | | Mannitol | |
|---|---|---|---|---|---|---|---|---|---|
| Weight (mg) | 0.25 | 1 | 5 | 20 | 1 | 1 | 5 | 1 | 20 |
| Mole ration versus phosphatidylcholine | 1 | 4 | 20 | 80 | 3 | 2 | 10 | 2 | 40 |
| Reaction mixture extract composition | | | | | | | | | |
| Phosphilipid derivative (III) | 3.2 | 27.9 | 9.6 | 2.8 | 15.3 | 39.6 | 21.8 | 11.6 | 13.0 |
| Phosphilipid derivative (IV) | Trace | 42.6 | 66.9 | 89.7 | 21.5 | 21.6 | 38.0 | 21.2 | 51.2 |
| Phosphatidic acid | 20.1 | 6.3 | 4.5 | Trace | 5.0 | 10.5 | 8.6 | 16.1 | 6.2 |
| Unreacted phospholipid derivative | 70.1 | 14.3 | 13.9 | 3.2 | 40.5 | 17.6 | 21.9 | 41.8 | 19.4 |
| Others | 6.6 | 9.4 | 5.1 | 4.3 | 10.0 | 10.6 | 9.7 | 9.3 | 10.1 |

* Erythritol

Example 2

To a suspension containing 2.7 g of the same soybean lecithin (Unimills Inc.) as that used in Example 1 as well as 240 ml of water there were added 60 ml of glycerin, 160 ml of 50 mM $CaCl_2$ solution, 265 ml of 20 mM acetate buffer (pH 5.5) and 5 ml of an enzyme solution containing 24 units of actinomycete-derived

phospholipase D. Then 10 ml of ethyl acetate was added, and the mixture was reacted at 50 °C for 60 minutes while shaking it, thereby producing 902 mg of cardiolipin.

When the reaction was effected in an otherwise identical manner except that no ethyl acetate was added the yield of cardiolipin was 695 mg.

Example 3

1470 µl of 50 mM $CaCl_2$ solution, 3897 µl of 20 mM acetate buffer (pH 5.5), 441 µl of glycerin and 73 µl of an enzyme solution containing 0.35 units of actinomycete-derived phospholipase D were added to 1470 µl of aqueous suspension containing 20 mg of a synthetic lecithin or dopalmitoyl phosphatidylcholine (Sigma Inc.) and were reacted at 45 °C for 2 hours while agitating the mixture. The desired product was then extracted with chloroform-methanol (2/1) and the extract was separated by silico-gel thin layer chromatography. The spots of the desired tetrapalmitoyl diphosphatidylglycerol were thereafter scraped off, and the extract liquid was vaporized and dried up, thereby producing 6 mg of the desired phospholipid derivative.

## Claims

1. A method of producing a phospholipid derivative having two phosphatidyl residues in its molecule, in which a phospholipid derivative of general formula:

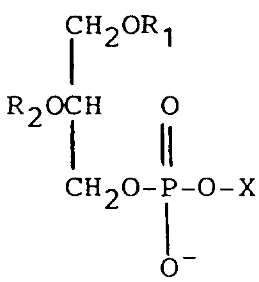

(I)

(in which $R_1$ and $R_2$, which may be the same or different, each represents an alkanoyl or an alkyl group, and X represents an organic residue derived by removal of one hydroxyl group from an hydroxyl-group-containing compound) is reacted with a polyhydric alcohol of general formula:

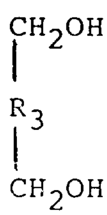

(II)

(in which $R_3$ represents an optionally-substituted alkylene group) in the presence of phospholipase D having transphosphatidylation activity, to produce a phospholipid derivative of general formula:

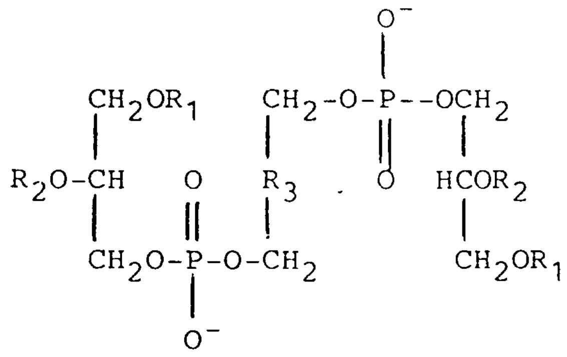

(III)

(in which $R_1$, $R_2$, $R_3$ and X all have the previously-stated meanings).

**2.** A method as claimed in claim 1, in which the alkanoyl or an alkyl group(s) $R_1$ and/or $R_2$ contains from 1 to 6 carbon atoms.

**3.** A method as claimed in claim 2, in which $R_1$ and/or $R_2$ contain(s) from 1 to 4 carbon atoms.

**4.** A method as claimed in claim 1, in which the alkylene group $R_3$ contains from 1 to 6 carbon atoms, and is optionally substituted with an hydroxyl group or with an alkoxy or hydroxy-alkyl group.

**5.** A method as claimed in claim 4, in which the alkylene group $R_3$ contains from 1 to 4 carbon atoms.

**6.** A method as claimed in claim 1, in which the alkoxy or hydroxy-alkyl substituents on the alkylene group $R_3$ contain from 1 to 6 carbon atoms.

**7.** A method as claimed in claim 6, in which $R_3$ is substituted with an alkoxy or hydroxy-alkyl group containing from 1 to 4 carbon atoms.

**8.** A method as claimed in any of the preceding claims, wherein the phospholipid derivative (I) is a natural lecithin, a phospholipid derivative fractioned from said natural lecithin, or a synthetic phospholipid derivative.

**9.** A method as claimed in claim 8, in which the natural lecithin is soybean lecithin, egg-yolk lecithin or rape-seed lecithin.

**10.** A method as claimed in any of the preceding claims, wherein the polyhydric alcohol (II) is or includes glycerin, erythritol, adonitol, mannitol, sorbitol, sedoheptylol and/or 2-hydroxymethyl-1, 3-propanediol.

**11.** A method as claimed in any of the preceding claims, in which the phospholipid derivative of general formula I is brought to reaction suspended in water.

**12.** A method as claimed in any of the preceding claims, in which the phospholipase D is introduced into a mixture of the other reactants and incubated therewith, if desired with agitation.

**13.** A method as claimed in claim 12, in which the transphosphatidylation activity of the phospholipase D is promoted by the presence of calcium ions.

**14.** A method as claimed in claim 13, in which the calcium ion is present at a concentration of from 1 to 100 mM.

**15.** A method as claimed in claim 14, in which the calcium ion concentration is in the range of from 4 to 30 mM.

**16.** A method as claimed in any of the preceding claims, in which the molar amount of polyhydric alcohol (II) employed is less than 50 times the molar amount of phospholipid derivative (I).

**17.** A method as claimed in claim 16, in which the molar amount of polyhydric alcohol (II) is from 2 to 40 times the molar amount of phospholipid derivative (I).

**18.** A method as claimed in any of the preceding claims, in which the phospholipase D employed is one derived from a streptomyces, and the reaction is effected at a temperature in the range of from 20° to 60°C and at a pH greater than 4.

**19.** A method as claimed in claim 18, in which the reaction is effected at a temperature of from 35° to 50°C.

**20.** A method as claimed in claim 18 or claim 19, in which the reaction is effected at a pH of from 5 to 8.4.

**21.** A method as claimed in any of the preceding claims, in which an amount of organic solvent is added to the reaction system sufficient to facilitate the dispersion of the phospholipid derivative (I) and active phospholipase D and thereby increase the reaction efficiency.

**22.** A method as claimed in claim 21, in which the organic solvent is or includes ethyl acetate added in an amount of from 1% to 6% (v/v).

**Patentansprüche**

**1.** Verfahren zum Herstellen eines Phospholipidderivats mit zwei Phosphatidylresten in seinem Molekül, bei welchem ein Phospholipidderivat der allgemeinen Formel

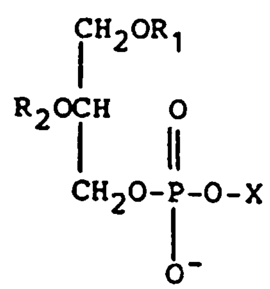

(I)

(in welcher $R_1$ und $R_2$, die gleich oder verschieden sein können, jeweils eine Alkanoyl- oder eine Alkylgruppe darstellen und X einen organischen Rest darstellt, der sich aus dem Entfernen einer Hydroxylgruppe aus einer hydroxylgruppenhaltigen Verbindung herleitet) mit einem mehrwertigen Alkohol der allgemeinen Formel

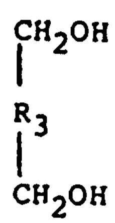

(II)

(in welcher $R_3$ eine gegebenenfalls substituierte Alkylengruppe darstellt) in Anwesenheit von Phospholipase D mit einer Transphosphatidylierungsaktivität unter Herstellen eines Phospholipidderivats der allgemeinen Formel

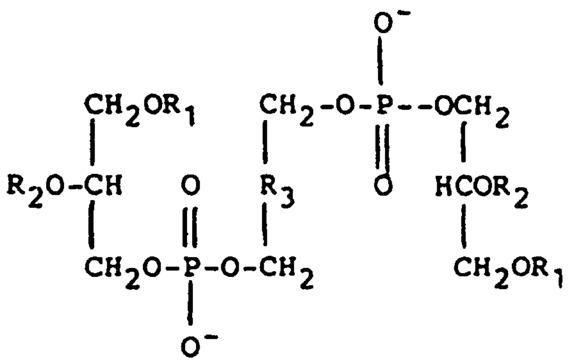

(III)

(worin $R_1$, $R_2$, $R_3$ und X alle die vorgenannten Bedeutungen besitzen) umgesetzt wird.

2. Verfahren wie in Anspruch 1 beansprucht, bei welchem die Alkanoyl- oder eine Alkylgruppe(n) $R_1$ und/oder $R_2$ 1 bis 6 Kohlenstoffatome enthält.

3. Verfahren wie in Anspruch 2 beansprucht, bei welchem $R_1$ und/oder $R_2$ 1 bis 4 Kohlenstoffatome enthält (enthalten).

4. Verfahren wie in Anspruch 1 beansprucht, bei welchem die Alkylengruppe $R_3$ 1 bis 6 Kohlenstoffatome enthält und gegebenenfalls mit einer Hydroxylgruppe oder mit einer Alkoxy- oder Hydroxyalkylgruppe substituiert ist.

5. Verfahren wie in Anspruch 4 beansprucht, bei welchem die Alkylengruppe $R_3$ 1 bis 4 Kohlenstoffatome enthält.

6. Verfahren wie in Anspruch 1 beansprucht, bei welchem die Alkoxy- oder Hydroxyalkylsubstituenten an der Alkylengruppe $R_3$ 1 bis 6 Kohlenstoffatome enthalten.

7. Verfahren wie in Anspruch 6 beansprucht, bei welchem $R_3$ mit einer Alkoxy- oder Hydroxyalkylgruppe substituiert ist, die 1 bis 4 Kohlenstoffatome enthält.

8. Verfahren wie in einem der vorangehenden Ansprüche beansprucht, bei welchem das Phospholipidderivat (I) ein natürliches Lezithin, ein aus dem natürlichen Lezithin fraktioniertes Phospholipidderivat oder ein synthetisches Phospholipidderivat ist.

9. Verfahren wie in Anspruch 8 beansprucht, bei welchem das natürliche Lezithin Sojabohnen-Lezithin, Eigelb-Lezithin oder Rapssamen-Lezithin ist.

10. Verfahren wie in einem der vorangehenden Ansprüche beansprucht, bei welchem der mehrwertige Alkohol (II) Glycerin, Erythrit, Adonit, Mannit, Sorbit, Sedoheptylol und/oder 2-Hydroxymethyl-1,3-propandiol ist oder einschließt.

11. Verfahren wie in einem der vorangehenden Ansprüche beansprucht, bei welchem das Phospholipidderivat der allgemeinen Formel I in Wasser suspendiert zur Reaktion gebracht wird.

12. Verfahren wie in einem der vorangehenden Ansprüche beansprucht, bei welchem die Phospholipase D in ein Gemisch der anderen Reaktionsteilnehmer eingebracht und damit gewünschtenfalls unter Rühren inkubiert wird.

13. Verfahren wie in Anspruch 12 beansprucht, bei welchem die Transphosphatidylierungsaktivität der Phospholipase D durch die Anwesenheit von Calciumionen gefördert wird.

14. Verfahren wie in Anspruch 13 beansprucht, bei welchem das Calciumion in einer Konzentration von 1 bis 100 mM vorhanden ist.

**15.** Verfahren wie in Anspruch 14 beansprucht, bei welchem das Calciumion in einer Konzentration von 4 bis 30 mM vorhanden ist.

**16.** Verfahren wie in einem der vorangehenden Ansprüche beansprucht, bei welchem die molare Menge des eingesetzten mehrwertigen Alkohols (II) geringer als die molare Menge Phospholipidderivat (I) ist.

**17.** Verfahren wie in Anspruch 16 beansprucht, bei welchem die molare Menge des mehrwertigen Alkohols (II) das 2- bis 40-fache der molaren Menge des Phospholipidderivats (I) beträgt.

**18.** Verfahren wie in einem der vorangehenden Ansprüche beansprucht, bei welchem die eingesetzte Phospholipase D eine von Streptomyces stammende ist und die Reaktion bei einer Temperatur im Bereich von 20° bis 60°C und bei einem größeren pH als 4 ausgeführt wird.

**19.** Verfahren wie in Anspruch 18 beansprucht, bei welchem die Reaktion bei einer Temperatur von 35° bis 50°C ausgeführt wird.

**20.** Verfahren wie in Anspruch 18 oder Anspruch 19 beansprucht, bei welchem die Reaktion bei einem pH von 5 bis 8,4 ausgeführt wird.

**21.** Verfahren wie in einem der vorangehenden Ansprüche beansprucht, bei welchem dem Reaktionssystem eine zum Erleichtern der Dispersion des Phospholipidderivats (I) und der aktiven Phospholipase D und dadurch dem Erhöhen der Reaktionsausbeute ausreichende Menge organisches Lösungsmittel zugesetzt wird.

**22.** Verfahren wie in Anspruch 21 beansprucht, bei welchem das organische Lösungsmittel Essigsäureethylester ist oder einschließt, welcher in einer Menge von 1% bis 6% (Vol./Vol.) zugesetzt wird.

**Revendications**

**1.** Procédé de production d'un dérivé de phospholipide possédant deux résidus phosphatidyle dans sa molécule, dans lequel on fait réagir un dérivé de phospholipide de formule générale :

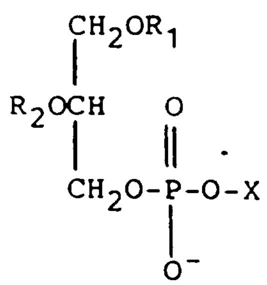

(I)

(dans laquelle $R_1$ et $R_2$, qui peuvent être identiques ou différents, représentent chacun un groupe alcanoyle ou alkyle, et X représente un résidu organique provenant de l'élimination d'un groupe hydroxyle d'un composé contenant le groupe hydroxyle) avec un alcool polyhydroxylé de formule générale :

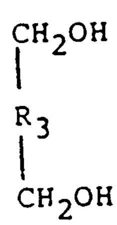

(II)

(dans laquelle $R_3$ représente un groupe alkylène éventuellement substitué), en présence d'une

phospholipase D ayant une activité de transphosphatidylation, pour produire un dérivé de phospholipide de formule générale

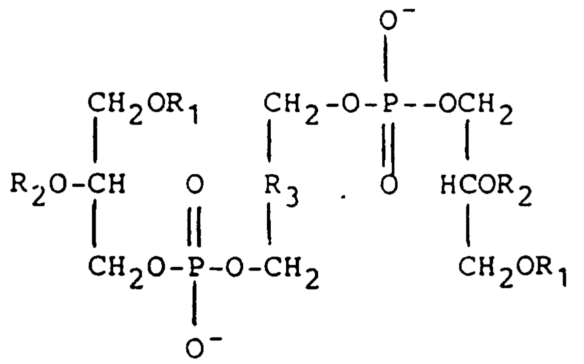

(III)

(dans laquelle $R_1$, $R_2$, $R_3$ et X ont tous les significations précédemment indiquées).

**2.** Procédé selon la revendication 1, dans lequel le(s) groupe(s) alcanoyle ou alkyle $R_1$ et/ou $R_2$ contiennent de 1 à 6 atomes de carbone.

**3.** Procédé selon la revendication 2, dans lequel $R_1$ et/ou $R_2$ contient ou contiennent de 1 à 4 atomes de carbone.

**4.** Procédé selon la revendication 1, dans lequel le groupe alkylène $R_3$ contient de 1 à 6 atomes de carbone, et est éventuellement substitué par un groupe hydroxyle ou par un groupe alcoxy ou hydroxyalkyle.

**5.** Procédé selon la revendication 4, dans lequel le groupe alkylène R3 contient de 1 à 4 atomes de carbone.

**6.** Procédé selon la revendication 1, dans lequel les substituants alcoxy ou hydroxyalkyle sur le groupe alkylène $R_3$ contiennent de 1 à 6 atomes de carbone.

**7.** Procédé selon la revendication 6, dans lequel R3 est substitué par un groupe alcoxy ou hydroxyalkyle contenant de 1 à 4 atomes de carbone.

**8.** Procédé selon l'une quelconque des revendications précédentes, dans lequel le dérivé de phospholipide (I) est une lécithine naturelle, un dérivé de phospholipide fractionné à partir de ladite lécithine naturelle ou un dérivé de phospholipide synthétique.

**9.** Procédé selon la revendication 8, dans lequel la lécithine naturelle est la lécithine de soja, la lécithine du jaune d'oeuf ou la lécithine du colza.

**10.** Procédé selon l'une quelconque des revendications précédentes, dans lequel l'alcool polyhydroxylé (II) est ou englobe la glycérine, l'érythritol, l'adonitol, le mannitol, le sorbitol, le sédoheptylol, et/ou le 2-hydroxyméthyl-1, 3-propanediol.

**11.** Procédé selon l'une quelconque des revendications précédentes, dans lequel le dérivé de phospholipide de formule générale I est mis en réaction, en suspension dans l'eau.

**12.** Procédé selon l'une quelconque des revendications précédentes, dans lequel la phospholipase D est introduite dans un mélange des autres corps réagissantset incubée avec eux, si nécessaire sous agitation.

**13.** Procédé selon la revendication 12, dans lequel l'activité de transphophatidylation de la phospholipase D est favorisée par la présence d'ions calcium.

**14.** Procédé selon la revendication 13, dans lequel l'ion calcium est présent à une concentration de 1 à 100 mM.

**15.** Procédé selon la revendication 14, dans lequel la concentration de l'ion calcium est comprise dans l'intervalle allant de 4 à 30 mM.

**16.** Procédé selon l'une quelconque des revendications précédentes, dans lequel la quantité molaire de l'alcool polyhydroxylé (II) utilisé est inférieure à 50 fois la quantité molaire du dérivé de phospholipide (I).

**17.** Procédé selon la revendication 16, dans lequel la quantité molaire de l'alcool polyhydroxylé (II) est de 2 à 40 fois la quantité molaire du dérivé phospholipide (I).

**18.** Procédé selon l'une quelconque des revendications précédentes, dans lequel la phospholipase D utilisée en est une qui provient d'un streptomyces, et la réaction s'effectue à une température comprise dans l'intervalle allant de 20° à 60° C et à un pH supérieur à 4.

**19.** Procédé selon la revendication 18, dans lequel la réaction s'effectue à une température allant de 35° à 50° C.

**20.** Procédé selon la revendication 18 ou la revendication 19, dans lequel la réaction s'effectue à un pH compris entre 5 et 8,4.

**21.** Procédé selon l'une quelconque des revendications précédentes, dans lequel on ajoute au système de réaction, une quantité de solvant organique suffisante pour faciliter la dispersion du dérivé de phospholipide (I) et de la phospholipase D active et on augmente ainsi l'efficacité de la réaction.

**22.** Procédé selon la revendication 21, dans lequel le solvant organique est ou englobe l'acétate d'éthyle ajouté en une quantité allant de 1 % à 6 % (vol/vol).